# EUROPEAN PATENT APPLICATION

(11) **EP 1 593 691 A1**
(43) Date of publication of application: **09.11.2005**
(21) Application number: 03774040.4
(22) Date of filing: 19.11.2003
(51) Int. Cl.: C07K 16/18

(54) **METHOD OF ACQUIRING HUMAN MONOCLONAL ANTIBODY INHIBITING CANCER CELL PROLIFERATION**

(30) Priority: 19.11.2002 JP 2002335281
(71) Applicant: Hagiwara, Hideaki, Tokyo 107-0052 (JP)
(72) Inventor: HAGIWARA, Yoshihide, (JP); HAGIWARA, Hideaki, Minato-ku Tokyo 107-0052 (JP)
(74) Representative: Hiebl, Inge Elisabeth
(86) International application number: PCT/JP2003/014697
(87) International publication number: WO 2004/046193

(57) **Abstract**

This invention provides a method of obtaining or screening human monoclonal antibodies or fragments thereof having cancer cell proliferation-inhibiting activity, **characterized by** contacting human monoclonal antibodies or fragments thereof with human vimentin or fragments thereof which at least include the region having the amino acid residues of Nos. 246 - 372 of the amino acid sequence of human vimentin, and selecting the human monoclonal antibodies or fragments thereof which specifically bind to the region having the amino acid residues of Nos. 246 - 372 of the amino acid sequence of human vimentin.

SEQUENCE LISTING
<110> HAGIWARA Yoshihide

<120> Method of Obtaining Human Monoclonal Antibodies
      Having Cancer Cell Proliferation-Inhibiting Activity

<130> K-27Hagi

<150> JP2002/335281
<151> 2002-11-19

<160> 4

<210> 1
<211> 24
<212> DNA
<213> Artificial Sequence

<220>
<223>Synthesized Referring to the Sequence Of Human Vimentin

<400> 1
tagcggccgc attctgaatc tcat                                          24

<210> 2
<211> 26
<212> DNA
<213> Artificial Sequence

<220>
<223> Synthesized Referring to the Sequence Of Human Vimentin

<400> 2
gcggccgcat cctgcaggcg gccaat                                        26

<210> 3
<211> 25
<212> DNA
<213> Artificial Sequence

<220>
<223> Synthesized Referring to the Sequence Of Human Vimentin

<400> 3
tagcggccgc catattctga atctc                                         25

<210> 4
<211> 24
<212> DNA
<213> Artificial Sequence

<220>
<223> Synthesized Referring to the Sequence Of Human Vimentin

<400> 4
ccagaattcc aggctcagat tcag                                                24

<210> 5
<211> 24
<212> DNA
<213> Artificial Sequence

<220>
<223> Synthesized Referring to the Sequence Of Human Vimentin

<400> 5
cgggaattcg aatggtacaa atcc                                                24

<210> 6
<211> 127
<212> PRT
<213> human

<220>
<223>

<400> 6
Gln Ala Gln Ile Gln Glu Gln His Val Gln Ile Asp Val Asp Val Ser
1               5                   10                  15
Lys Pro Asp Leu Thr Ala Ala Leu Arg Asp Val Arg Gln Gln Tyr Glu
            20                  25                  30
Ser Val Ala Ala Lys Asn Leu Gln Glu Ala Glu Glu Trp Tyr Lys Ser
        35                  40                  45
Lys Phe Ala Asp Leu Ser Glu Ala Ala Asn Arg Asn Asn Asp Ala Leu
    50                  55                  60
Arg Gln Ala Lys Gln Glu Ser Thr Glu Tyr Arg Arg Gln Val Gln Ser
65                  70                  75                  80
Leu Thr Cys Glu Val Asp Ala Leu Lys Gly Thr Asn Glu Ser Leu Glu
                85                  90                  95
Arg Gln Met Arg Glu Met Glu Glu Asn Phe Ala Val Glu Ala Ala Asn
            100                 105                 110

Tyr Gln Asp Thr Ile Gly Arg Leu Gln Asp Glu Ile Gln Asn Met
        115                 120                 125

## Description

### Technical Field

This invention relates to a method of obtaining or screening human monoclonal antibodies or their fragments which exhibit cancer cell proliferation- inhibiting activity and are broadly useful in medical and pharmaceutical fields for, e.g., therapy, diagnosis and prophylactic treatments; and pharmacological and biochemical fields for biochemical reagents, purified reagents for biological polymers and the like.

### Background Art

Cancer cells are thought to have cancer-specific antigens or cancer-related antigens which are qualitatively and quantitatively different from those of normal cells, and in certain cases cancer cells having such antigens are excluded by the host's immunity system. During early researches in human cancers, in blood serum of cancer patients presence of antibodies which react with their own cancer cells was confirmed. This gave rise to an anticipation that such antibodies, if could stably be supplied in large quantities, would be of very high utilization value in therapy and diagnoses of cancer. However, there existed such problems that 1) blood serum of cancer patients contained antibodies of very versatile specificities and isolation of particular antibodies which react with specific auto-cancer cells was difficult; 2) such antibodies were quantitatively limited; 3) their stable supply could not be secured; and the like.

Monoclonal antibody-producing technology using hybridoma method, which was developed by Kohler and Milstein solved these problems. After their report, many mouse monoclonal antibodies which would react with human cancer cells were produced by immunizing mice with human cancer cells or extracts thereof, and antigens which the antibodies recognized were identified. Also clinical application of a part of the antibodies was attempted.

One drawback which has become clear from the clinical tests, however, is that frequent administration to a man of mouse-derived antibodies which are heterologous to human induces HAMA reaction (Human Anti-mouse antibody response) and in consequence causes side-action and deterioration in therapeutic effect. Hence, production of isogenic anti-cancer antibodies of higher safety, i.e., human anti-cancer monoclonal antibodies has been desired.

Under the circumstances, as disclosed in detail in JP Hei 1 (1989)-59878B, Hei 7 (1995)-121221B, Hei 7 (1995)-119240B, Japanese Patent Nos. 2,599,258 and 2,509,191, JP Hei 8 (1996)-29078B, Hei 7 (1995)-98000B, Japanese Patent Nos. 2,721,817 and 2,830,976, JP Sho 62 (1987)-70400A, JP Hei 6 (1994)-141,884A and JP Hei 9 (1997)-100300A, we created various human-human hybridomas by means of cell fusion of lymphocytes from cancer patients with human lymphoblast B-cell and obtained many human monocolonal antibodies having binding affinity to cancer cells. We have conducted further studies on the antibodies to discover that no clear correlation exists between the cancer cell-binding activity and anti-cancer effect (cell proliferation-inhibiting effect) of human monoclonal antibodies, i.e., not all of the antibodies which bind to cancer cells exhibit anti-cancer effect. Specificities of antibodies which bind to cancer cells are very versatile, and it is virtually impossible to examine anti-cancer effect of all of them.

### Disclosure of The Invention

The main object of the present invention is to provide a simple and convenient method which enables screening and production of antibodies which exhibit anti-cancer effect, out of those many human monoclonal antibodies which bind to cancer cells.

With the view to solve the above problem, we conducted the following experiments.

Previously, we created by means of cell fusion of lymphocytes from patient of cervical carcinoma with human lymphoblast B-cell, human-human hybridoma cell line CLNH11 (ATCC HB8307) which produced human monoclonal antibodies of high reactivity with human cancer cells. Furthermore, we made it clear that the monoclonal antibody, CLN-IgG, which is produced by CLNH11, binds to not only cervical carcinoma cells but also many other kinds of cancer cells such as brain tumor, lung cancer, stomach cancer and colorectal cancer, and inhibits proliferation of cancer cells. We also made it clear that the antigen which CLN-IgG recognizes is human vimentin which is one of cytoskeletal proteins (Hagiwara, et al., Human Antibodies 10,77-82 (2001)). We prepared various fragments of human vimentin to examine binding affinity of each of the fragments with CLN-IgG and identified the antigen epitope region which was recognized by CLN-IgG to find that the epitope was present in the region having the amino acid residues of Nos. 289 to 367 on human vimentin's rod C2 domain (cf. JP 2002-51785A).

In the occasion of producing antibodies expecting their effect of inhibiting cancer cell proliferation, customarily cell proliferation-relating proteins (e.g., cell proliferation factor receptors on cell membrane or cell proliferation factors), in particular, cell surface proteins excessively expressed in cancer cells, were used as target antigens. Therefore, it was entirely unknown that human vimentin which is a cytoskeletal protein functions as a cancer antigen and at the same time can serve as a target for proliferation-inhibiting activity of antibodies. Naturally, there was absolutely no anti-cancer human monoclonal antibody targeting human vimentin existed.

We then investigated relevancy between specificity of various human monoclonal antibodies which react with human cancer cells and their anti-tumor effectivity. Consequently, we now ascertained: antibodies which bind to a specific epitope on human vimentin possess cancer cell proliferation-inhibiting activity, while those which do not have that binding affinity show substantially no cancer cell proliferation-inhibiting activity. This led us to discover that the human monoclonal antibodies or fragments thereof which have the activities of destroying cancer cells or of inhibiting their proliferation could be selected and obtained, by selecting monoclonal antibodies or fragments thereof which specifically bind to human vimentin or human vimentin fragments including at least the region having the amino acid residues of Nos. 246 - 372 in the amino acid sequence of human vimentin. The present invention is whereby completed.

Thus, according to the invention a method for obtaining or screening human monoclonal antibodies or fragments thereof which have cancer cell proliferation-inhibiting activity is provided, said method comprising contacting human monoclonal antibodies or fragments thereof with human vimentin or a human vimentin fragment including at least the region having the amino acid sesidues of Nos. 246 - 372 in the amino acid sequence of the human vimentin, and screening the human monoclonal antibodies or fragments thereof which specifically bind to the region having the amino acid residues of Nos. 246 - 372 in the amino acid sequence of human vimentin.

### Brief Explanation of Drawings

Fig. 1 is a chart showing binding affinity between human monoclonal antibody and vimentin of human glioblastoma cell line, U-251MG, by means of Western blotting. In Fig. 1,
1: molecular weight marker
2: CLN-IgG
3:TOH/G2-IgG
4:IM9-IgG
5:HT2-IgM.

In the drawing, the arrow at the right side indicates the position of human vimentin.

Fig. 2 are charts showing binding affinity between the given human monoclonal antibodies and vimentin fragment-GST fusion protein, by means of Western blotting. In Fig. 2,

### A. Western blotting using CLN-IgG

1: molecular weight maker
2: GST
3: human vimentin C2 domain (amino acid residues of Nos. 246 - 397)-GST fusion protein
4: human vimentin C2 fragment (amino acid residues of Nos. 246 - 367)-GST fusion protein
5: human vimentin C2 fragment (amino acid residues of Nos. 246 - 372)-GST fusion protein
6: human vimentin C2 fragment (amino acid residues of Nos. 289 - 367)-GST fusion protein
7: human vimentin C2 fragment (amino acid residues of Nos. 246 - 371)-GST fusion protein
8: human vimentin C2 fragment (amino acid residues of Nos. 246 - 372)-GST fusion protein
9: human vimentin C2 fragment (amino acid residues of Nos. 289 - 371)-GST fusion protein
10: human vimentin C2 fragment (amino acid residues of Nos. 289 - 372)-GST fusion protein
11: molecular weight marker

### B. Western blotting using HT-2 IgM

Lanes 1 - 6 were CBB stained; lanes 7 - 12 were expressed by Western blotting.
1: molecular weight marker
2: human vimentin C1 domain (amino acid residues of Nos. 96 - 245)-GST fusion protein
3: human vimentin C2 domain (amino acid residues of Nos. 246 - 397)-GST fusion protein
4: human vimentin C2 fragment (amino acid residues of Nos. 246 - 372)-GST fusion protein
5: BSA (bovine serum albumin)
6: human glioblastomas U-251 (MG cell extract)
7: molecular weight marker
8: human vimentin C1 domain (amino acid residues of Nos. 96 - 245)-GST fusion protein
9: human vimentin C2 domain (amino acid residues of Nos. 246 - 397)-GST fusion protein
10: human vimentin C2 fragment (amino acid residues of Nos. 246 - 372)-GST fusion protein
11: BSA (bovine serum albumin)
12: human glioblastomas U-251 (MG cell extract)

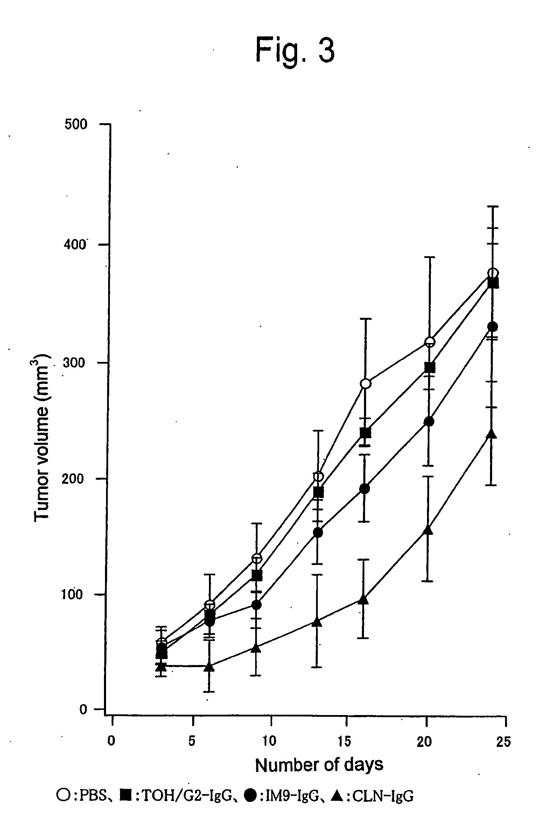

Fig. 3 is a graph illustrating cell proliferation-inhibiting effect of various human monoclonal antibodies against nude mouse-implanted cancer (human cervical carcinoma cell line ME-180), in which
- ○ :: PBS
- ■ :: TOH/G2-IgG
- • :: IM9-IgG
- ▲ :: CLN-IgG.

Hereafter the method which is provided by the present invention is explained in further details.

### Detailed Description of The Invention

Human vimentin is a protein (amino acid residue number: 466; molecular weight: 53,651) which is classified under medium diameter filament, among those cytoskeletal proteins which act to maintain cell structures. Human vimentin is constructed of four domains of, from its N-terminal side, head, coil 1 (C1), coil 2 (C2) and tail, among which C1 and C2 in particular have spiral structures.

An epitope which is recognized by human monoclonal antibody CLN-IgG is present on the C2 domain. With the view to more accurately identify this epitope region, we incorporated DNAs which encode various C2 fragmetns into E. coli. expression vectors to cause their expression as GST (glutatione S transferase) fusion proteins and purified the same. Thereafter we investigated binding affinity between the fragments and monoclonal antibodies, by Western blotting and ELISA. In consequence, it has become clear that two kinds of monoclonal antibodies bind to vimentin fragment at a region having the amino acid residues of Nos. 289 - 367 (cf. JP 2002-51785A).

For investigating cancer cell proliferation-inhibiting activity of various human monoclonal antibodies, then we conducted an in vivo test using nude mice. First a human monoclonal antibody and a cervical carcinoma cell line ME-180 were mixed and hypodermically implanted in nude mice. Subsequently, resulted tumor volumes were measured with passage of time to evaluate individual antibody's cell proliferation-inhibiting effect. In consequence, only with the antibodies which had binding affinity to the above-specified human vimentin epitope exhibited potent cell proliferation-inhibiting effect.

From the foregoing, it is demonstrated that there exists an intimate relationship between binding affinity of human monoclonal antibodies to the epitope region on C2 domain of human vimentin and their cancer cell proliferation-inhibiting effect. Accordingly, it is possible to select and obtain with ease those human monoclonal antibodies which exhibit cancer cell proliferation-inhibiting effect, with use of the human vimentin epitope.

Concerning the epitope region on C2 domain of human vimentin recognized by human monoclonal antibody CLN-IgG, we previously made it clear that said region corresponds to that of the amino acid residues of Nos. 289 - 367 in the amino acid sequence of human vimentin, by the steps of: producing varieties of peptide fragments of human vimentin's C2 domain; identifying the antigen epitope region recognized by CLN-IgG based on reactivity of each of the fragments with CLN-IgG; and analyzing the amino acid sequence of that particular region (cf. JP-2002-51785A).

In consequence of further investigations in the epitope region on C2 domain of human vimentin, which is recognized by human monoclonal antibody CLN-IgG, including its three-dimensional configuration, we noticed a possibility that CLN-IgG recognizes a still broader region than the antigen epitope region having the amino acid sequence of human vimentin. Analyzing the amino acid sequence of such a broader region, we have determined the region having amino acid residues of Nos. 246 - 372 of the amino acid sequence of human vimentin, as below (Sequence No. 6 in the sequence list):

Gln Ala Gln Ile Gln Glu Gln His Val Gln Ile Asp Val Asp Val Ser
246 250 260
Lys Pro Asp Leu Thr Ala Ala Leu Arg Asp Val Arg Gln Gln Tyr Glu
270
Ser Val Ala Ala Lys Asn Leu Gln Glu Ala Glu Glu Trp Tyr Lys Ser
280 290

Lys Phe Ala Asp Leu Ser Glu Ala Ala Asn Arg Asn Asn Asp Ala Leu
300
Arg Gln Ala Lys Gln Glu Ser Thr Glu Tyr Arg Arg Gln Val Gln Ser
310 320
Leu Thr Cys Glu Val Asp Ala Leu Lys Gly Thr Asn Glu Ser Leu Glu
330 340
Arg Gln Met Arg Glu Met Glu Glu Asn Phe Ala Val Glu Ala Ala Asn
350
Tyr Gln Asp Thr Ile Gly Arg Leu Gln Asp Glu Ile Gln Asn Met
360 370 372

Above human vimentin fragment including the region including amino acid residues of Nos. 246 - 372 of the amino acid sequence (which may be hereafter referred to as "human vimentin epitope fragment") can be produced by a number of methods. For example, the human vimentin epitope fragment can be chemically synthesized by means of solid phase or liquid phase syntheses which are known per se. It can also be produced by genetic engineering technology, comprising the steps of synthesizing from the amino acid sequence of object human vimentin epitope fragment a DNA encoding the same, and applying it to a vector-host cell system formed of a host such as bacteria, animal cells, plant cells or the like and an expression vector therefor. In the latter case, the fragment can also be expressed in the form of fusion protein having various functions.

Human vimentin epitope fragments are subject to no specific limitation about their size, so long as they are bindable to CLN-IgG. They include those whose amino acid sequences are partially deleted, replaced and/or added within the scope not impairing their binding affinity to CLN-IgG.

On the other hand, according to the method of the present invention, human cells expressing human vimetnin, e.g., human glioblastoma cell line U-251 MG per se, can be used as human vimentin. Crude or purified human vimentins which are separated from above cells following ordinary protein purification means, for example, an affinity chromatography, may also be used.

As methods for screening and aquiring human monoclonal antibody or a fragment thereof (hereafter may be collectively referred to as "human monoclonal antibody" for convenience) having cancer cell proliferation-inhibiting activity, using human vimentin or human vimentin epitope fragment, for example, those comprising immobilizing human vimentin or human vimentin epitope fragment on an adequate solid carrier (e.g., microplate, nitrocellulose membrane, nylon membrane, glass beads, resin, sensor chips and the like); contacting the same with a liquid containing a test human monoclonal antibody or a fragment thereof; and detecting human monoclonal antibodies or fragments thereof on the carrier which bind to the human vimentin or human vimentin epitope fragment, by such means as Western blotting which utilizes oxygen antibody method, ELISA, dot blotting, assays utilizing surface plasmon resonance; or the like.

Thus those human monoclonal antibodies or fragments thereof which specifically bind to the region having amino acid residues of Nos. 246 - 372 of amino acid sequence of human vimentin can be selected and acquired.

As the test human monoclonal antibodies, not only purifed human monoclonal antibodies but also the cells themselves which are producing human monoclonal antibodies can be used.

Those human monoclonal antibodies or fragments thereof thus detected or acquired according to the method of the present invention possess the effect of inhibiting proliferation of various cancer cells in correspondence to their origin and are expected to be useful as active ingredients of cancer cell proliferation-inhibiting agents, for treating cancerous diseases such as uterine cancer, lung cancer, stomach cancer, colorectal cancer, brain tumor, liver cancer, breast cancer, prostate cancer and the like.

In clinical use of the human monoclonal antibodies or fragments thereof which are detected or acquired according to the invention as cancer cell proliferation-inhibiting agent, the human monoclonal antibodies or fragments thereof can be formulated into a preparation form of, for example, lyophilized powder together with any suitable excipient by the means known per se. So obtained preparation can be reconstituted with distilled water for injection and parenterally administered into, for example, a patient's vein or tumor.

### Examples

Hereafter the present invention is explained in further details referring to working Examples, it being understood that the invention is in no way thereby restricted.

### Example 1:

Selection of human monoclonal antibody binding to human vimentin by Western blotting

Human glioblastoma cell line U-251 MG (Human Science Foundation IFO 50288) was ultrasonically homogenized in a solution containing 62.5 mM Tris (pH 6.8), 2% SDS, 5% 2-mercaptoethanol and 4M urea. Resulting homogenate corresponding to 5 × 10⁴ cells was subjected to SDS-polyacrylamide gel electrophoresis (SDS-PAGE).

After the electrophoresis, the proteins were transferred to Hybond ECL membranes (Amersham Pharmacia Biotech, Inc.) using a semi-dry type blotting machine, in 48 Mm Tris, 39 mM glycine, 0.037% SDS and 10% methanol.

The membranes after the transfer were immersed in a blocking solution (PBS-T containing 5% skim milk (phosphate buffered physiological saline containing 0.3% Tween 20)) at 37°C for an hour and thereafter immersed in 10 µg/mL of primary antibody solutions (CLN-IgG, TOH/G2-IgG, IM9-IgG or HT2-IgM, each as dissolved in PBS-T containing 1% skim milk) at 37°C for 30 minutes.

The membranes were further washed three times with PBS-T, and immersed in secondary antibody solutions (peroxidase-labeled affinity purified goat antihuman IgG antibody or peroxidase-labeled affinity purified goat antihuman IgM antibody, as dissolved with PBS-T containing 1% skim milk (both are products of BIOSORCE Co., diluted by 10,000X)) at 37°C for 30 minutes. After washing the membranes with PBS-T three times, ECL detection reagent (Amersham Pharmacia Biotech, Inc.) was added, followed by 1 minute's standing (ECL detection reagent is a high sensitivity system which detects the target antigen by chemoluminescence using the peroxidase-labeled antibody, from the proteins immobilized on the membranes).

Finally the detection reagent was removed from the membranes, and the membranes were interposed between OHP sheets and exposed to X-ray film for one minute. The films were then developed.

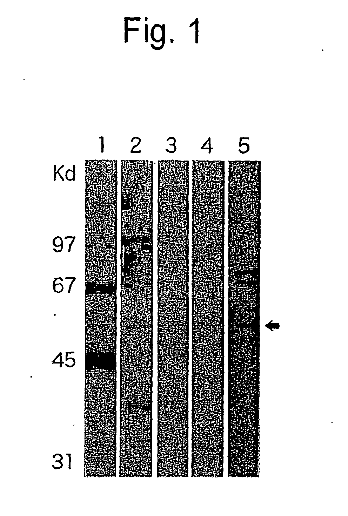

The result was, as shown in Fig. 1, of the four tested human monoclonal antibodies, two of CLN-IgG and HT2-IgM were found to recognize human vimentin (in Fig. 1, the arrow shows the position of the vimentin).

### Example 2:

### Production of human vimentin C2 fragment-GST fusion protein

Based on the amino acid sequence of human vimentin, 3'-end DNA primers of sequence Nos. 1, 2 and 3, and 5'- end DNA primers of sequence Nos. 4 and 5, as shown in the sequence list, were synthesized. PCR was carried out by combining these primers in various ways and using the plasmid including human vimentin C2 domain as template, to amplify various partial sequences on C2 domain. So obtained fragments were digested with EcoRI and NotI and linked with GST fusion protein expression vector pGEX-4T-1 (Amersham Pharmacia Biotech, Inc.) which had been digested with EcoRI and NotI using DNA Ligation Kit, Version 2 (Takara Shuzo Co.). E. coli BL 21 (Amersham BioScience) was transformed with the resulted plasmid, and ampicillin-resistant strain was selected, from which plasmids were prepared by alkali process. These plasmids were digested with EcoRI and NotI and given an agarose electrophoresis to allow identification and selection of clones in which human vimentin C2 fragment was inserted.

Those E. coli clones having each of the recombinant plasmids as obtained above were inoculated into 2mL LB medium (bacto trypton, 10 g/L; bacto yeast extract, 5 g/L; sodium chloride, 10 g/L) and cultured at 25°C for an overnight. Each of the culture liquids was inoculated into 20mL of LB medium again, and cultured at 25°C for 3.5 hours. Then 2 µL of 1M IPTG (isopropyl-β-D-thiogalactopyranoside) was added and the cultivation was continued for further 2 hours. The culture was then centrifuged at 5,000 rpm for 5 minutes to collect the cells. The supernatant was discarded and 1mL of PBS (phosphate-buffered saline, physiological saline (pH 7.2)) was added to the precipitate. The liquid in which the cells were suspended was given an ultrasonic treatment, followed by addition of 1 mL of 20% TritonX-100, an hour's shaking at 4°C and purification over affinity chromatography using glutathione cephalose 4B (Pharmacia Co.), to provide a human vimentin C2 fragment-GST fusion protein.

### Example 3:

### Selection of human monoclonal antibody binding to human vimentin C2 fragment by means of Western blotting

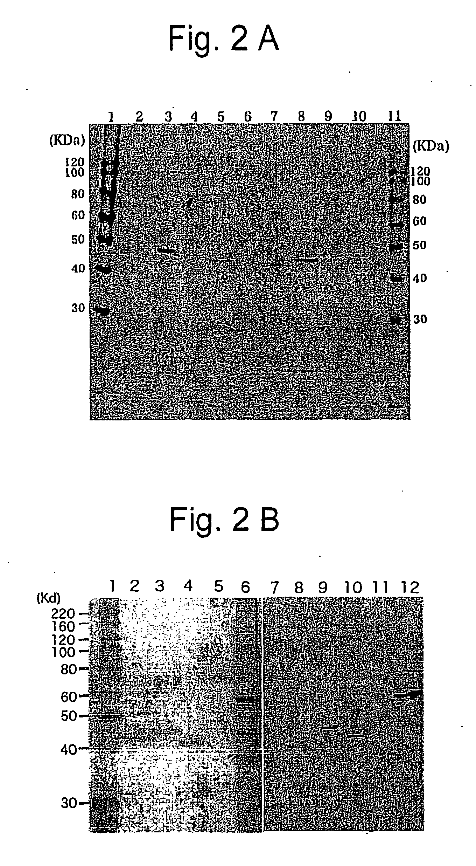

Each of the fusion proteins as obtained in Example 1 was adjusted to a concentration value of 200 ng/lane and SDS-PAGE was carried out. From the gels after the electrophoresis, the proteins were transferred onto Hybond-ECL membranes (Amersham Pharmacia Biothech, Inc.) by means of semi-dry blotting. The membranes after the transfer were immersed in a blocking solution (PBS-T containing 5% skim milk) at 37°C for an hour, and then immersed in primary antibody solutions (CLN-IgG or HT2-IgM, dissolved with PBS-T containng 1% skim milk (each 10µg/mL)) at 37°C for 30 minutes. The membranes were washed with PBS-T three times, and immersed is secondary antibody solutions (peroxidase-labeled affinity purified goat anti-human IgG antibody or peroxidase-labeled affinity purified goat anti-human IgM antibody, as dissolved with 1% skim milk-containing PBS-T (both are products of BIOSORCE Co., diluted by 25,000X)) at 37°C for 30 minutes. The memberanes were washed with PBS-T three times, and to which then ECL detection reagent (Amersham Pharmacia Biotech, Inc.) was added, followed by a minute's standing. The detection reagent was removed from the membranes, which were then interposed between OHPsheets and exposed to X-ray film for 1 minute. The films were then developed. The results are shown in Fig. 2. In Fig. 2B, besides the fusion proteins, bovine serum albumin and cell extract (homogenized cell solution in 8M urea, 2%SDS, 4% DTT and 0.125 M Tris-HCl at pH 6.8) of glioblastoma cell line U-251 MG were given the electrophoresis as controls, at the concentration of 200 ng/lane and 10 µg protein/lane, respectively.

From the results it was found that both CLN-IgG (Fig. 2A) and HT2-IgM antibodies (fig. 2B) reacted with human vimentin (amino acid residues of Nos. 246 - 397), in particular, reacted strongly with human vimentin C2 epitope fragment (amino acid residues of Nos. 246 -372). From this fact it has become clear that CLN-IgG and HT2-IgM recognize the region having amino acid residues of Nos. 246 - 372 of human vimentin.

### Example 4:

### Cancer cell proliferation-inhibiting effect on nude mouse-implanted cancer

Human cervical carcinoma ME-180 cell line (ATCC HTB33) 5 × 10⁶ were mixed with 170 µg of human monoclonal antibodies (CLN-IgG (ATCC HB8307), TOH/G2-IgG or IM9-IgG (ATCC CCL159)) and hypodermically implanted into nude mice (Clea Japan, Inc., BALB/cA JC1-nu nu/nu 6 weeks old female, 5 mice/group) and resulting tumor volumes were measured at regular time intervals. The tumor volume was determined following an approximate expression of (large diameter) × (shorter diameter)² × 1/2.

In consequence, with IM9-IgG and TOH/G2-IgG which are not binding to human vimentin or human vimentin epitope nearly no cancer cell proliferation-inhibiting effect was recognized. By contrast, CLN-IgG having binding affinity to human vimentin or human vimentin epitope exhibited potent cancer cell proliferation-inhibiting effect (Fig. 3).

### Example 5:

### In vitro cancer cell proliferation-inhibiting test

To a DF medium containng 10% fetal bovine serum (FBS), 100 µL each of human glioblastoma cell line U251MG suspension at a concentration of 5 × 10⁴/mL was dispersed into a 96-well microplate (Nunc Co). Into each of the microplates HT2-IgM or IM9-IgG (ATCC CCL159) was added such that the ultimate concentration reached 50 µg/mL or 100 µg/mL. After two days' incubation under the conditions of 5% carbon dioxide gas concentration at 37°C, BrdU uptake by the S-stage cells was measured using Cell Proliferation ELISA reagent (Boehringer Mannheim). More specifically, 10 µL per well of BrdU (100 µM) was added, cultured for 2 hours, cells were immobilized, DNAs were denatured, reacted with peroxidase-labeled anti-BrdU antibodies for 90 minutes, and in the last a substrate, tetramethylbenzidine (TMB), was added and absorbance at 370 nm was measured. By means of comparing with the control groups composed of only the cells to which no antibody was added, the antibodies' cell proliferation-inhibiting activity was determined.

The results were as shown in the following table (TABLE 1). HT2-IgM exhibited cancer cell proliferation-inhibiting effect, while IM9-IgG did not. That is, HT2-IgM antibody which has binding affinity to human vimentin epitope fragment exhibited the proliferation-inhibiting effect, while IM9-IgG antibody which has none of the binding affinity did not exhibit the proliferation-inhibiting effect. This fact renders it possible to select and acquire antibodies having cell proliferation-inhibiting effect, by examining presence or absence of candidate antibodies' reactivity with the epitope fragment.

**TABLE 1:**

| in vitro Cancer Cell Proliferation-inhibiting effect of human monoclonal antibody | | |
|---|---|---|
| Human monoclonal antibody | Concentration (µg/mL) | Cell proliferation-inhibiting activity (%)^{a} |
| HT2-IgM | 50 | 28.7 |
| | 100 | 38.4 |
| IM9-IgG | 50 | -1.4 |
| | 100 | -20.0 |

| | | |
|---|---|---|
| a. Proliferation-inhibiting activity determined by using as the control the groups composed of only the cells to which no human monoclonal antibody was added. | | |

## Claims

1. A method of obtaining or screening human monoclonal antibodies or fragments thereof which possess cancer cell proliferation-inhibiting activity, **characterized by** comprising contacting human monoclonal antibodies or fragments thereof with human vimentin or a human vimentin fragment which includes at least the region having amino acid residues of Nos. 246 - 372 of the amino acid sequence of human vimentin, and selecting the human monoclonal antibodies or fragments thereof which specifically bind to the region having amino acid residues of Nos. 246 - 372 of the amino acid sequence of human vimentin.

2. Human monoclonal antibodies which are obtained by the method as described in Claim 1.

3. A fragment derived from a human monoclonal antibody which is obtained by the method as described in Claim 1, which specifically binds to the region having amino acid residues of Nos. 246 - 372 of amino acid sequence of human vimentin.

4. A cancer cell proliferation-inhibiting agent **characterized by** containing as the active ingredient a human monoclonal antibody or fragment thereof which specifically bind to the region having amino acid residues of Nos. 246 - 372 in the amino acid sequence of human vimentin.

5. A pharmaceutical preparation which comprises a human monoclonal antibody or fragment thereof which specifically bind to the region having amino acid residues of Nos. 246 -372 in the amino acid sequence of human vimentin, and pharmaceutically acceptable carrier.

6. A method for suppressing proliferation of cancer cells which comprises administering human monoclonal antibody or fragment thereof which specifically bind to the region having amino acid residues of Nos. 246 - 372 in the amino acid sequence of human vimentin.
